# EUROPEAN PATENT APPLICATION

(11) **EP 4 714 574 A1**
(43) Date of publication of application: **25.03.2026**
(21) Application number: 24830438.8
(22) Date of filing: 06.06.2024
(51) Int. Cl.: B22F 1/102, C22C 27/04, A61L 31/10, A61L 31/18, C22C 1/05, B22F 1/142, B22F 3/24, B22F 3/10, B23P 15/00, A61L 31/02

(54) **MOLYBDENUM-BASED ALLOY AND PREPARATION METHOD THEREFOR, AND MEDICAL IMPLANT DEVICE AND PREPARATION METHOD THEREFOR**

(30) Priority: 29.06.2023 CN 202310787723
(71) Applicant: Shanghai MicroPort Medical (Group) Co., Ltd., Shanghai 201203 (CN)
(72) Inventor: WANG, Jiaojiao, Shanghai 201203 (CN); LIU, Zixin, Shanghai 201203 (CN); ZHOU, Qi, Shanghai 201203 (CN)
(74) Representative: Marks & Clerk LLP
(86) International application number: PCT/CN2024/097650
(87) International publication number: WO 2025/001792

(57) **Abstract**

The present invention provides a molybdenum-based alloy, a method of preparing the alloy, a medical implant device and a preparation method therefor. The molybdenum-based alloy includes a molybdenum matrix, polydopamine and alloying element. The alloying element includes(s) at least one of strengthening and toughening alloying element and purifying alloying element. The strengthening and toughening alloying element includes(s) at least one of tantalum, iridium and platinum. The purifying alloying element includes(s) at least one of strontium, titanium and cerium. The medical implant device is made of the molybdenum-based alloy. The invention overcomes the problems of room-temperature embrittlement and anisotropy associated with conventional molybdenum-based alloys, and the molybdenum-based alloy that it provides exhibits improved overall performance, which ensures that vascular stents and other medical implant devices made of the alloy exhibit excellent mechanical properties when used at in-vivo temperatures.

## Description

### TECHNICAL FIELD

The present invention relates to the preparation of molybdenum-based alloys and, in particular, to a molybdenum-based alloy, a method of preparing the molybdenum-based alloy, a medical implant device and a method of preparing the implant.

### BACKGROUND

Coronary heart disease (CHD) is known to be a disease that occurs commonly and frequently. The incidence of CHD has been rising year by year, and this disease has been recognized as one of the major diseases that threaten the physical and mental health of the Chinese people. At present, the most important therapy for cardiovascular diseases is stent-based minimally invasive intervention. Most existing stents are non-degradable, permanent metal ones, the use of which, however, tends to cause intimal hyperplasia, restenosis, later thrombosis or the like due to continual mechanical stress, foreign body inflammatory responses and their other drawbacks. Such limitations of conventional metal stents can be overcome by the use of a degradable metal stent, which, when deployed in a lumen of a blood vessel to provide support, will gradually degrade and eventually disappear after the blood vessel is remodeled. Degradable stents have been recognized as a trend for future vascular stents.

Degradable vascular stents currently under studies include metal (Mg, Zn, Fe) and polymer ones, and there have been made available degradable magnesium alloy and polymer stent products. However, all these degradable stent products have been found with problems that are difficult to solve. For example, magnesium alloys cannot ensure adequate mechanical integrity over three months due to too rapid degradation, while iron alloys cannot degrade completely due to too slow degradation. Although zinc corrodes and degrades at a rate compatible with clinical needs (20 µm/y), it shows poor mechanical properties and is associated with the issues of room-temperature aging and work softening. In terms of mechanical properties, magnesium alloys are low in strength and inferior in plasticity. Degradable polymer materials have even poorer mechanical properties than metal materials, and tend to exhibit anisotropy. In terms of biosafety, magnesium alloys degrade too fast and, when implanted, will lead to an undesirably high pH and the release of gaseous hydrogen, which may cause severe inflammatory reactions. For iron alloys, there is still no reliable evidence on the safety of rust that they produce from their degradation. In terms of X-ray radiopacity, magnesium alloys and polymers are not ideal due to their inherent disadvantages. This increases surgical complexity and risk and creates inconvenience in postoperative follow-up.

Molybdenum is one of the trace elements essential for human nutrition, and recommended daily intake of this element for adults is 0.15-0.5 mg. As an important component of numerous enzymes, molybdenum participates in iron utilization in the human body, prevents anemia, promotes physical development and growth and aids in the metabolism of carbohydrates and fats. Moreover, it maintains adequate myocardial energy metabolism and prevents Keshan disease. It also maintains arterial elasticity and prevents cardiovascular disease. Further, it supports the human immune system. Studies of Fraunhofer IFAM in Germany and the University of Michigan in the United States of America have demonstrated that molybdenum is uniformly degradable at a moderate rate comparable to that of zinc alloys. In addition, molybdenum does not affect cellular activity or cause thrombosis or inflammatory reactions. Further, in vitro and in vivo studies in animals have demonstrated good biosafety of molybdenum.

However, molybdenum and its alloys inherently exhibit a range of undesirable processing characteristics, including a high deformation temperature, easy oxidation at high temperatures, high tensile strength, poor plasticity, a rapid cooling rate and surges in tensile strength in response to temperature drops. Such high deformation resistance of molybdenum and its alloys have significantly limited their use in the field of medical devices. In particular, low-temperature embrittlement and poor cross-sectional mechanical properties of molybdenum and existing molybdenum-based alloys are challenging the use of these materials for the fabrication of medical devices intended for use around room temperature. Therefore, there is a need for developing a novel, high-strength, high-toughness, degradable, molybdenum-based alloy for medical use around room temperature.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY

It is an object of the present invention to provide a molybdenum-based alloy, a method of preparing the alloy, a medical implant device and a preparation method therefor, which overcome one or more of the above described problems.

The above object is attained by a molybdenum-based alloy proposed herein, which includes a molybdenum matrix, polydopamine and an alloying element, the alloying element including at least one of strengthening and toughening alloying element and purifying alloying element, the strengthening and toughening alloying element including at least one of tantalum, iridium and platinum, the purifying alloying element including at least one of strontium, titanium and cerium.

Optionally, a mass percentage of the strengthening and toughening alloying element in the molybdenum-based alloy may be less than or equal to 20%.

Optionally, the mass percentage of the strengthening and toughening alloying element in the molybdenum-based alloy may be 0.5% to 2%.

Optionally, a mass percentage of the purifying alloying element in the molybdenum-based alloy may be less than or equal to 1.0%.

Optionally, the mass percentage of the purifying alloying element in the molybdenum-based alloy may be 0.01% to 0.04%.

Optionally, a mass percentage of the polydopamine in the molybdenum-based alloy may be less than or equal to 1.5%.

Optionally, the mass percentage of the polydopamine in the molybdenum-based alloy may be 0.1% to 0.3%.

Optionally, a mass percentage of an impurity element in the molybdenum-based alloy may be less than or equal to 0.2%, wherein the impurity element includes at least one of chromium, copper, iron, nickel, tungsten, manganese, silicon, oxygen, nitrogen, carbon and hydrogen.

Optionally, the mass percentage of copper, iron and nickel in the molybdenum-based alloy may be each less than 20 ppm, the mass percentage of carbon is less than or equal to 0.03%; the mass percentages of hydrogen, oxygen, and nitrogen are each less than or equal to 0.02%; and the mass percentages of chromium, silicon, tungsten, and manganese are each less than or equal to 0.05%.

Optionally, the molybdenum-based alloy may do not contain any of calcium, magnesium, sodium, sulfur, lead, bismuth, zinc, rhenium, yttrium, zirconium and tin.

The above object is also attained by a method of preparing a molybdenum-based alloy, according to which the molybdenum-based alloy as defined above is obtainable. The method includes:
blending a raw material of a molybdenum matrix with a raw material of an alloying element in proportion, obtaining an alloy raw material, the raw material of the alloying element including at least one of a raw material of the strengthening and toughening alloying element and a raw material of the purifying alloying element, the raw material of the strengthening and toughening alloying element including at least one of a raw material of tantalum, a raw material of iridium and a raw material of platinum, the raw material of the purifying alloying element including at least one of a raw material of strontium, a raw material of titanium and a raw material of cerium;
adding polydopamine to the alloy raw material, subjecting the alloy raw material added with the polydopamine to an ingot melting and casting process or a powder metallurgy process and thereby obtaining a preform of the molybdenum-based alloy; and
strengthening and toughening the preform and thereby obtaining the molybdenum-based alloy.

Optionally, the raw material of the molybdenum matrix, the raw material of the strengthening and toughening alloying element and the raw material of the purifying alloying element may be all in the form of powders, wherein:
subjecting the raw material of the alloy added with the polydopamine to the powder metallurgy process and thereby obtaining the preform of the molybdenum-based alloy includes:
uniformly mixing the raw material of the alloy added with the polydopamine, obtaining a mixed alloy powder;
pressing the mixed alloy powder, obtaining a mixed alloy compact; and
sintering the mixed alloy compact, thereby obtaining the preform of the molybdenum-based alloy.

Optionally, before the polydopamine is added to the raw material of the alloy, the method may further include:
subjecting the raw material of the alloy to an annealing process in a reducing atmosphere or under a vacuum condition.

Optionally, the polydopamine may be able to polymerize on surfaces of particles in the powder of the raw material of the alloy, forming a polydopamine nano-coating with a thickness in the range of from 10 nanometers to 60 nanometers.

Optionally, strengthening and toughening the preform and thereby obtaining the molybdenum-based alloy may include:
subjecting the preform of the molybdenum-based alloy successively to solid solution strengthening, hot deformation and annealing, thereby obtaining the molybdenum-based alloy.

The above object is also attained by a medical implant device proposed herein, which is made of the molybdenum-based alloy as defined above.

The above object is also attained by a method of preparing a medical implant device, which includes:
obtaining a molybdenum-based alloy according to the method as defined above;
performing a first process on the molybdenum-based alloy and thereby obtaining a tubular blank of the molybdenum-based alloy;
performing a second process on the tubular blank of the molybdenum-based alloy and thereby obtaining a preform of the medical implant device; and
post-processing the preform of the medical implant device and thereby obtaining the medical implant device.

Optionally, performing the first process on the molybdenum-based alloy and thereby obtaining the tubular blank of the molybdenum-based alloy may include:
subjecting the molybdenum-based alloy to hot piercing, hot extrusion or mechanical piercing, thereby obtaining the tubular blank of the molybdenum-based alloy.

Optionally, performing the second process on the tubular blank of the molybdenum-based alloy and thereby obtaining the preform of the medical implant device may include:
subjecting the tubular blank of the molybdenum-based alloy successively to drawing, stress relief annealing and laser cutting, thereby obtaining the preform of the medical implant device.

Optionally, post-processing the preform of the medical implant device and thereby obtaining the medical implant device may include:
subjecting the preform of the medical implant device successively to pickling, heat treatment and polishing, thereby obtaining the medical implant device.

The proposed molybdenum-based alloy, medical implant device and methods offer the following advantages over the prior art:
The proposed molybdenum-based alloy includes a molybdenum matrix, polydopamine and alloying element(s), the alloying element(s) including at least one of strengthening and toughening alloying element(s) and purifying alloying element(s), the strengthening and toughening alloying element(s) including at least one of tantalum, iridium and platinum, the purifying alloying element(s) including at least one of strontium, titanium and cerium. The presence of polydopamine in the molybdenum-based alloy ensures sufficient surface adsorption and good surface adhesion of particles in a powder for making the alloy and provides good lubrication, addressing the problems of inadequate material compactness and uniformity that may otherwise arise in the preparation of the molybdenum-based alloy. Adding the strengthening and toughening alloying element(s), such as tantalum, iridium and/or platinum, to the molybdenum-based alloy can effectively reduce yield strength of the resulting alloy, leading to an increase in its plasticity. Additionally, in doing so, the molybdenum-based alloy has an increased recrystallization temperature and behaves better at higher temperatures. The presence of the strengthening and toughening alloying element(s), such as tantalum, iridium and/or platinum, in the molybdenum-based alloy improves its room-temperature behaviors, weldability and radiation-shielding properties and fundamentally addresses the problems of room-temperature embrittlement and anisotropy, enhancing its overall performance. This ensures that vascular stents and other medical implant devices made of the proposed molybdenum-based alloy exhibit excellent mechanical properties when used at in-vivo temperatures. Through adding the purifying alloying element(s) such as strontium, titanium and/or cerium to the molybdenum-based alloy, the presence of the interstitial impurities C, N and O is reduced, enhancing the prevention of the alloy's embrittlement and anisotropy. Moreover, this strengthens and toughens the molybdenum matrix, ensuring desired transverse elongation properties of a vascular stent or another medical implant device made of the alloy for post-dilation during use. Further, the proposed molybdenum-based alloy also provides excellent corrosion resistance, degradability and radiopacity. Therefore, this alloy can be used to make various medical implant devices with high strength, high toughness and consistent performance for use in a wider range of medical and clinical applications.

Since the first method can be used to prepare the proposed molybdenum-based alloy, it offers all the benefits of the alloy. For details, reference is made to the above description in connection with the benefits of the proposed molybdenum-based alloy, and these are not repeated herein. According to this method, strengthening and toughening the preform of the molybdenum-based alloy can impart additionally improved mechanical properties to the resulting molybdenum-based alloy, effectively ensuring that the resulting molybdenum-based alloy exhibits high strength and high toughness.

Because of being made of the proposed molybdenum-based alloy, the inventive medical implant device provides the advantages of high strength, high toughness and consistent performance and is uniformly degradable, ensuring biosafety during use. In addition, this medical implant device exhibits excellent radiopacity, which brings convenience to surgeons' operation and clinical practice.

Since the second method is based on the same inventive concept as the first method, it offers all the benefits of the latter. For details, reference is made to the above description in connection with the benefits of the first method, and these are not repeated herein.

### BRIEF DESCRIPTION OF THE DRAWINGS

Fig. 1 is a schematic diagram illustrating a corrosion resistance comparison made between vascular stents made of a molybdenum-based alloy prepared in accordance with the present invention and a cobalt-based alloy.
Fig. 2a is an SEM image showing the surface morphology of a sheet of a molybdenum-based alloy according to the present invention, which has undergone uniform degradation.
Fig. 2b is an SEM image showing the surface morphology of a wire of a molybdenum-based alloy according to the present invention, which has undergone uniform degradation.
Fig. 3 shows a schematic flowchart of a method of preparing a molybdenum-based alloy according to an embodiment of the present invention.
Fig. 4 shows a schematic flowchart of a method of preparing a medical implant device according to an embodiment of the present invention.

### DETAILED DESCRIPTION

Molybdenum-based alloys, methods of preparing a molybdenum-based alloy, medical implant devices and methods of preparing a medical implant device according to specific embodiments disclosed herein will be described in greater detail below with reference to the accompanying drawings. From the following description, advantages and features of the present invention will become more apparent. Note that the figures are provided in a very simplified form not necessarily drawn to exact scale for the only purpose of helping to explain the disclosed embodiments in a more convenient and clearer way. In order that the objects, features and advantages of the present invention can be more apparent and readily understood, reference is to be made to the accompanying drawings. It would be recognized that architectural, proportional, dimensional and other details in the figures are presented only for the purpose of facilitating, in conjunction with the disclosure herein, the understanding and reading of those familiar with the art, rather than being intended to limit conditions under which the present invention can be implemented. Therefore, any and all architectural modifications, proportional variations or dimensional changes that achieve the same or similar benefits and objects as the present invention are considered to fall within the scope of the teachings herein. The specific design features of the invention as disclosed herein, including, for example, specific dimensions, orientations, locations and shapes, will be determined in part by the particular intended application and use environment. Additionally, in the embodiments described below, like reference numerals may be sometimes used to refer to the same or functionally similar elements throughout different figures, while description thereof may not be repeated. In this specification, similar reference numerals and letters refer to similar items in the figures, and thus once an item is defined in one figure, it may not be discussed for following figures. Further, if a method is described herein as including a series of steps, the order of these steps as presented herein is not necessarily the only order in which they can be performed, and some of the stated steps may be omitted and/or other steps not described herein may be added to the method.

It is to be noted that relational terms such as "first," "second," and the like may be used herein solely to distinguish one entity or action from another entity or action, without necessarily requiring or implying any actual such relationship or order between such entities or actions, or denoting or implying relative importance of them, or implicitly indicating the number of them. Moreover, the terms "comprise", "include" and any variations thereof are intended to cover a non-exclusive inclusion, such that a process, method, article, or apparatus that includes a list of elements is not necessarily limited to those elements, but may include other elements not expressly listed or inherent to such process, method, article, or apparatus. An element preceded by "comprises... a" does not, without more constraints, preclude the existence of additional identical elements in the process, method, article, or apparatus that comprises the element. As used herein, the singular forms "a", "an" and "the" include plural referents, and the term "or" is generally employed in the sense of "and/or", "a number of" of "at least one", and "at least two" of "two or more".

It should be noted that reference throughout this specification to "one embodiment", "some embodiments", "an example", "a specific example" "some examples" or the like means that a particular feature, structure, material, or characteristic described in connection with the embodiments or examples is included in at least one embodiment or example of the invention. Thus, the appearances of those phrases in various places throughout this specification are not necessarily referring to the same embodiment or example of the invention. Furthermore, the particular features, structures, materials, or characteristics may be combined in any suitable manner in any one or more embodiments. Further, should there be no contradiction, one of ordinary skill in the art can combine the various embodiments, examples and features thereof described herein in any combination.

Before describing the alloys, implants and methods proposed herein in detail, relevant characteristics of molybdenum and alloys thereof will be briefed below, for the purpose of facilitating an understanding of the present invention.

Tensile strength of pure molybdenum is close to that of 316L, and it degrades at a moderate rate comparable to that of Zn. Moreover, pure molybdenum has a density of 10.2 g/cm³ and shows much superior radiopacity to cobalt-based and degradable materials (iron-based, magnesium-based, zinc-based and polymeric). It also exhibits compatibility with magnetic resonance imaging (MRI). Compared with the several existing degradable biomaterials, degradable molybdenum and its alloys offer significant advantages in mechanical properties, degradability, biosafety and X-ray radiopacity. Therefore, they are currently considered as the most ideal and promising candidates for the next-generation degradable metal stents that can address key bottlenecks in clinical use of such materials.

Molybdenum is a typical brittle refractory metal and melts at a temperature as high as 2,620 °C, second only to tungsten, rhenium and tantalum. This metal and its alloys possess excellent high-temperature strength, good corrosion resistance and other unique mechanical and chemical properties. These high-temperature refractory metals offer excellent overall performance and meet very strict requirements for use under high-temperature conditions. However, the toughness of molybdenum and its alloys varies as a function of temperature over only a very narrow range before experiencing a ductile-to-brittle fracture transition. The ductile-to-brittle transition temperature of pure molybdenum is in the range of about 140 to 150 °C. Consequently, this metal and its alloys are difficult to further process, resulting in low product performance and a limited range of application.

It is a principal object of the present invention to provide a molybdenum-based alloy, a method of preparing the alloy, a medical implant device and a preparation method therefor, which overcome the problems with molybdenum and known alloys thereof arising from a range of their inherent undesirable processing characteristics, including a high deformation temperature, easy oxidation at high temperatures, high tensile strength, poor plasticity, a rapid cooling rate and surges in tensile strength in response to temperature drops. Such high resistance of the metal and alloys to deformation has significantly limited their use in the field of medical devices. In particular, their low-temperature embrittlement and poor cross-sectional mechanical properties are seriously challenging their use for the fabrication of medical devices intended for use around room temperature.

To this end, the present invention provides a molybdenum-based alloy including a molybdenum matrix, polydopamine and alloying element(s). The alloying element(s) include(s) at least one of strengthening and toughening alloying element(s) and purifying alloying element(s). The strengthening and toughening alloying element(s) include(s) at least one of tantalum, iridium and platinum. The purifying alloying element(s) include(s) at least one of strontium, titanium and cerium. The polydopamine added to the molybdenum-based alloy ensures sufficient surface adsorption and good surface adhesion of particles in a powder for preparing the alloy and provides good lubrication, addressing the problems of inadequate material compactness and uniformity that may otherwise arise in the preparation of the molybdenum-based alloy. Adding the strengthening and toughening alloying element(s), such as tantalum, iridium and/or platinum, to the molybdenum-based alloy can effectively reduce yield strength of the resulting alloy, leading to an increase in its plasticity. Additionally, in doing so, the molybdenum-based alloy has an increased recrystallization temperature and behaves better at higher temperatures. The presence of the strengthening and toughening alloying element(s), such as tantalum, iridium and/or platinum, in the molybdenum-based alloy improves its room-temperature behaviors, weldability and radiation-shielding properties and fundamentally addresses the problems of room-temperature embrittlement and anisotropy, enhancing its overall performance. This ensures that vascular stents and other medical implant devices made of the proposed molybdenum-based alloy exhibit excellent mechanical properties when used at in-vivo temperatures. Through adding the purifying alloying element(s) such as strontium, titanium and/or cerium to the molybdenum-based alloy, the presence of the interstitial impurities C, N and O is reduced, enhancing the prevention of the alloy's embrittlement and anisotropy. Moreover, this strengthens and toughens the molybdenum matrix, ensuring desired transverse elongation properties of a vascular stent or another medical implant device made of the alloy for post-dilation during use. Further, the proposed molybdenum-based alloy also provides excellent corrosion resistance, degradability and radiopacity. Therefore, this alloy can be used to make various medical implant devices with high strength, high toughness and consistent performance for use in a wider range of medical and clinical applications.

Performance benefits from the presence of the strengthening and toughening element(s) are explained in detail below.

Specifically, any element with more s- and d-orbital electrons than molybdenum (Mo) added to the molybdenum-based alloy can have a remarkable softening effect thereon. As noted above, suitable examples of the strengthening and toughening alloying element(s) according to the present invention include tantalum (Ta), iridium (Ir) and platinum (Pt), the numbers of s- and d-orbital electrons of which are 43, 47 and 49, respectively, all greater than that of Mo (14). Notably, the numbers of s-orbital electrons of Ta, Ir and Pt are all odd. Adding any of these elements to the molybdenum matrix can mitigate directional atomic bonding induced by the asymmetric d-orbital electron distribution of pure molybdenum in its second outermost shell. Both Ta and Ir have two outermost electrons, which are distributed symmetrically. The outermost s-orbital electrons of Pt are distributed in spherical symmetry and all exhibit metallic bonding characteristics. For these reasons, adding the strengthening and toughening alloying element(s) (at least one of Ta, Ir and Pt) to the molybdenum matrix can remarkably improve the latter's ductile-brittle fracture behavior and reduce anisotropy of the molybdenum-based alloy. Consequently, the molybdenum-based alloy has a significantly reduced ductile-to-brittle transition temperature, and this decreases as the presence of the strengthening and toughening alloying element(s) increases. For example, the ductile-to-brittle transition temperature may even drop to as low as approximately -200 °C at a content of 50% of the strengthening and toughening alloying element(s). The presence of the strengthening and toughening alloying element(s) also raises a recrystallization temperature of the molybdenum-based alloy, enhances its high-temperature performance, and improves its room-temperature performance, weldability and radiation-shielding properties. Thus, adding the strengthening and toughening alloying element(s) fundamentally addresses the problems of room-temperature embrittlement and anisotropy associated with molybdenum, enhancing the overall performance of the molybdenum-based alloy.

The presence of the strengthening and toughening alloying element(s) (at least one of Ta, Ir and Pt) at a certain level in the molybdenum-based alloy can impart excellent room-temperature processability. Possible reasons for this are considered to be the three facts: (1) the strengthening and toughening alloying element(s) react(s) with molybdenum to produce a compound of the MoXO₄ type without grain boundary infiltration, but not of the MoO₂ type; (2) the strengthening and toughening alloying element(s) increase(s) the solubility of C and O, making carbides and oxides difficult to precipitate and avoiding segregation of these impurity elements at grain boundaries; and (3) unlike pure molybdenum metal, the molybdenum-based alloy containing the strengthening and toughening alloying element(s) undergoes twinning deformation. The strengthening and toughening alloying element(s) modify(ies) the electron distribution of molybdenum, thus reducing its directionality of atomic bonding, inhibiting its shift from metallic bonding to covalent bonding, lowering its stacking fault energy and increasing its shear modulus. Therefore, this provides a fundamental approach to the nature of low-temperature embrittlement of Mo.

Further, since each of Ta, Ir and Pt has a high density, adding Ta, Ir and/or Pt can additionally enhance radiopacity of the molybdenum-based alloy. It should be noted and will be appreciated by those skilled in the art that even without the addition of those elements, the molybdenum-based alloy will still exhibit remarkably superior radiopacity to the degradable materials currently available on the market.

In some exemplary embodiments, the strengthening and toughening alloying element(s) is/are present in the molybdenum-based alloy at a percentage of 20% or less by weight. With this composition, the strengthening and toughening alloying element(s) is/are present in an amount sufficient to ensure elimination of room-temperature embrittlement and anisotropy of the molybdenum-based alloy, improving overall performance thereof. Moreover, this amount can avoid excessive presence of the strengthening and toughening alloying element(s), which may have an adverse impact on degradability of the molybdenum-based alloy.

Further, the strengthening and toughening alloying element(s) may be present in the molybdenum-based alloy at a percentage of 15% or less by weight. Further, the strengthening and toughening alloying element(s) may be present in the molybdenum-based alloy at a percentage of 10% or less by weight. Further, the strengthening and toughening alloying element(s) may be present in the molybdenum-based alloy at a percentage of 8% or less by weight. Further, the strengthening and toughening alloying element(s) may be present in the molybdenum-based alloy at a percentage of 6% or less by weight. Further, the strengthening and toughening alloying element(s) may be present in the molybdenum-based alloy at a percentage of 3% or less by weight. Further, the strengthening and toughening alloying element(s) may be present in the molybdenum-based alloy at a percentage of 0.5% to 2% by weight.

Performance benefits from the presence of the purifying element(s) are explained in detail below.

Specifically, another import factor that affects the plasticity and embrittlement of pure molybdenum is the presence of impurity elements therein. Additionally, interstitial impurities affecting the brittleness of molybdenum are mainly C, N and O, among others. The molybdenum metal is very susceptible to segregation of these interstitial atoms at grain boundaries. Oxygen readily reacts with other elements, and the resulting oxides segregate at grain boundaries and within grains, hindering grain boundary sliding and considerably affecting the plasticity of molybdenum. Nitrogen (N), even when present in a minor amount, will greatly raise the risk of intergranular embrittlement of molybdenum. The presence of carbon (C) in a moderate amount at grain boundaries can mitigate the embrittlement of molybdenum. However, an excess of carbon present at grain boundaries will lead to large coarse carbide precipitates, which are highly detrimental to the plasticity of molybdenum. The H/C atomic ratio is another factor that affects the embrittlement of molybdenum. If C/O<2, the plasticity of molybdenum will be inferior due to a high oxygen content. However, if C/O>2, carbides will precipitate, strengthening grain boundaries of molybdenum and mitigating its embrittlement.

Generally speaking, the presence of N in the polycrystalline molybdenum material leads to a higher ductile-to-brittle transition temperature and enhanced embrittlement thereof. In order to avoid enrichment of the aforementioned interstitial impurities at grain boundaries, the purifying alloying element(s), such as Sr, Ti and/or Ce, may be added in accordance with the present invention. The purifying alloying element(s) react(s) with the interstitial impurities C, N and O, forming fine, high-melting-point carbide particles dispersed across the matrix. These particles provide dispersion strengthening, grain refining and dislocation hindering effects, greatly enhancing room-temperature and high-temperature mechanical properties of the molybdenum matrix. On the one hand, when the purifying alloying element(s) (at least one of Sr, Ti and Ce) is/are present in a moderate amount, the compounds produced from its/their reactions with the interstitial impurities C, N and O bond strongly to the molybdenum matrix and are mostly present as precipitates at Mo grain boundaries. This reduces the driving force toward segregation of C, N and O atoms at grain boundaries, leading to a lower ductile-to-brittle transition temperature. On the other hand, the compounds produced from the reactions of the purifying alloying element(s), such as Sr, Ti and/or Ce, with the interstitial impurities C, N and O can effectively strengthen interfaces with weak bonding in the polycrystalline molybdenum matrix, decrease the tendency of intergranular brittle fracture and reduce anisotropy, thereby improving the plasticity of the polycrystalline molybdenum-based alloy. In addition, during preparation of the molybdenum-based alloy, the purifying alloying element(s), such as Sr, Ti and/or Ce, may be added in the form of a powder consisting of nanoparticles, which coat the surfaces of large particles of the major component(s) (molybdenum, and optionally the strengthening and toughening alloying element(s), if present), so that they generally resemble "sesame balls". Providing heterogeneous nucleation sites, the nanoparticles effectively promote uniform growth of equiaxed crystals, eliminating the cracking issue associated with growth into dendritic grains. In particular, this ensures desired transverse elongation properties of a medical implant device (e.g., stent) made of the alloy for post-dilation during use. Therefore, the molybdenum-based alloy can be used to make various medical implant devices with high strength, high toughness and consistent performance for use in a wider range of medical and clinical applications.

In some exemplary embodiments, the purifying alloying element(s) is/are present in the molybdenum-based alloy at a percentage of 1.0% or less by weight. With this composition, the purifying alloying element(s) is/are present in an amount sufficient to ensure mitigation of embrittlement and anisotropy of the molybdenum-based alloy and strengthening and toughening of the molybdenum matrix. Moreover, this amount ensures that, during preparation of the molybdenum-based alloy, the purifying alloying element(s) can be added in the form of a powder consisting of nanoparticles, which coat the surfaces of large particles of the major component(s) (molybdenum, and optionally the strengthening and toughening alloying element(s), if present), so that they generally resemble "sesame balls". In this way, the nanoparticles provide heterogeneous nucleation sites, effectively promoting uniform growth of equiaxed crystals and eliminating the cracking issue associated with growth into dendritic grains.

Further, the purifying alloying element(s) may be present in the molybdenum-based alloy at a percentage of 0.8% or less by weight. Further, the purifying alloying element(s) may be present in the molybdenum-based alloy at a percentage of 0.5% or less by weight. Further, the purifying alloying element(s) may be present in the molybdenum-based alloy at a percentage of 0.2% or less by weight. Further, the purifying alloying element(s) may be present in the molybdenum-based alloy at a percentage of 0.1% or less by weight. Further, the purifying alloying element(s) may be present in the molybdenum-based alloy at a percentage of 0.06% or less by weight. Further, the purifying alloying element(s) may be present in the molybdenum-based alloy at a percentage of 0.01% to 0.04% or less by weight.

Performance benefits from the presence of the polydopamine are explained in detail below.

Specifically, by virtue of its exceptional adhesive properties, the polydopamine (PDA) can coat particles in powders of the metals (including the molybdenum matrix, the strengthening and toughening alloying element(s) and the purifying alloying element(s)), forming a uniform, dense nano-coating with even higher adsorption. This can prevent oxidation and impurities and enhance cohesion of particles in the powders. The PDA also provides lubrication during molding of the powders. As a strong ligand for polyvalent metal ions, such as Sr, Ti and Ce ions, the PDA can enhance cohesion of particles in the powders, providing for obtaining of a texturally uniform, highly dense material for the molybdenum-based alloy.

Adding the PDA to a molding agent for powder metallurgy can offer the advantages of wetting the metal powders so as to make them easier to compact and enabling desirable uniform dispersion of particle agglomerates across the metal powders, which allows for the agglomerates to be more easily broken up. Thus, a more uniform texture and density can be obtained. Usually, a mixture of the metal powders and the molding agent is compacted to increase their degree of mixing and remove air. This enables powder metallurgy to achieve an even denser texture.

Since the PDA-based molding agent would suffice as long as it can coat solid particles at a thickness on the order of nanometers, the PDA does not need to be present in a large amount. In some exemplary embodiments, the PDA is present in the molybdenum-based alloy at a percentage of 1.5% or less by weight. Further, the PDA may be present in the molybdenum-based alloy at a percentage of 1.2% or less by weight. Further, the PDA may be present in the molybdenum-based alloy at a percentage of 1% or less by weight. Further, the PDA may be present in the molybdenum-based alloy at a percentage of 0.7% or less by weight. Further, the PDA may be present in the molybdenum-based alloy at a percentage of 0.4% or less by weight. Further, the PDA may be present in the molybdenum-based alloy at a percentage of 0.1% to 0.3% by weight.

In some exemplary embodiments, the molybdenum-based alloy contains 0.2% or less by weight of impurity elements, at least one of chromium, copper, iron, nickel, tungsten, manganese, silicon, oxygen, nitrogen, carbon and hydrogen. In particular, the presence of these impurity elements may be due to the inevitable introduction of some impurities, such as other metals (than the molybdenum matrix, strengthening and toughening alloying element(s) and purifying alloying element(s)), half-metals, metal salts and/or non-metals, during preparation of the molybdenum-based alloy. As these impurity elements would affect the plasticity and processability of the molybdenum-based alloy, the species and presence of them must be strictly controlled.

The impurity elements may be present in the molybdenum-based alloy at a percentage of 0.15% or less, preferably 0.1% or less, more preferably 0.08% or less, even more preferably 0.06% or less, most preferably 0.03% or less, by weight.

In some exemplary embodiments, in the molybdenum-based alloy, carbon is present at most at a percentage by weight of 0.03% (i.e., 0.03% or less), and each of hydrogen, oxygen and nitrogen of 0.02% (i.e., 0.02% or less). Further, each of the other impurity elements may be present in the molybdenum-based alloy at a percentage by weight not exceeding 0.05% (i.e., 0.05% or less), preferably not exceeding 0.03% (i.e., 0.03% or less), more preferably less than 0.01%, even more preferably less than 80 parts per million (ppm), still even more preferably less than 50 ppm, particularly preferably less than 20 ppm.

The presence of calcium (Ca) and magnesium (Mg) is extremely detrimental to the plasticity of the molybdenum-based alloy. If the molybdenum-based alloy contains the two elements, it is expected to have dramatically degraded plasticity, because the presence of them may lead to "blistering" during molybdenum blank sintering and extremely large coarse grains resulting from recrystallization annealing. Moreover, the fact that Ca and Mg usually appear at grain boundaries itself is very unfavorable to the plasticity of the molybdenum-based alloy. The presence of some other elements, such as iron (Fe) and nickel (Ni), may degrade the strength of the molybdenum-based alloy when it is processed at a high temperature and lower its recrystallization temperature. Moreover, during pressing, cracks often develop from locations with Fe and Ni. Sodium (Na) and copper (Cu), when present in the matrix, will significantly affect the processability of the molybdenum-based alloy due to their immiscibility with Mo. Sulfur (S) is considered to be an extremely detrimental impurity element because it reacts with other impurities to form eutectic sulfides in the matrix at grain boundaries, making a blank of the molybdenum-based alloy exhibit thermal embrittlement. Additionally, the molybdenum-based alloy, when processed, will exhibit thermal embrittlement if it contains a low-melting-point element such as lead (Pb), bismuth (Bi) or tin (Sn). For these reasons, raw materials for the proposed molybdenum-based alloy are preferably absent of any one of Ca, Mg, Fe, Ni, Cu, Na, S, Pb, Bi and Sn. Further, the raw materials may also not contain any one of zinc (Zn), rhenium (Re), yttrium (Y) and zirconium (Zr).

Reference is now made to Tables 1 and 2 below. Table 1 shows the compositions of molybdenum-based alloys prepared in accordance with Examples 1 to 8 of the present invention and of molybdenum and molybdenum-based alloys prepared in accordance with Comparative Examples 1 to 5. Table 2 summarizes the results of performance tests performed on the molybdenum and molybdenum-based alloys.

**Table 1 Compositions of Molybdenum-Based Alloys**

| Composition (wt%) | Ta | Pt | Ir | Sr | Ti | Ce | Zr | Ca | Fe | PDA | Mo |
|---|---|---|---|---|---|---|---|---|---|---|---|
| Example 1 | 20 | 0 | 0 | 0 | 0 | 0 | 0 | 0 | 0 | 0.1 | balance |
| Example 2 | 0 | 15 | 0 | 0.03 | 0 | 0.05 | 0 | 0 | 0 | 0.3 | balance |
| Example 3 | 0 | 0 | 10 | 0 | 0.06 | 0 | 0 | 0 | 0 | 0.4 | balance |
| Example 4 | 8 | 0 | 0 | 0 | 0 | 0.1 | 0 | 0 | 0 | 0.7 | balance |
| Example 5 | 0 | 6 | 0 | 0.2 | 0 | 0 | 0 | 0 | 0 | 1 | balance |
| Example 6 | 1.2 | 0 | 3 | 0 | 0.5 | 0 | 0 | 0 | 0 | 1.2 | balance |
| Example 7 | 0 | 1 | 0 | 0 | 0 | 0.8 | 0 | 0 | 0 | 1.5 | balance |
| Example 8 | 0 | 0 | 0 | 1 | 0 | 0 | 0 | 0 | 0 | 0.9 | balance |
| Comparative Example 1 | 0 | 0 | 0 | 0 | 0 | 0 | 0 | 0 | 0 | 0 | balance |
| Comparative Example 2 | 0 | 0 | 0 | 0 | 0.4 | 0 | 0.08 | 0 | 0 | 0 | balance |
| Comparative Example 3 | 0 | 0 | 0 | 0 | 0 | 0 | 0 | 0.03 | 0 | 0 | balance |
| Comparative Example 4 | 0 | 0 | 0 | 0 | 0 | 0 | 0 | 0 | 0.05 | 0 | balance |
| Comparative Example 5 | 0 | 0 | 10 | 0 | 0.06 | 0 | 0 | 0 | 0 | 0 | balance |

**Table 2 Results of Performance Tests**

| Mechanical Properties | Elastic Modulus (GPa) | Density (g/cm³) | Longitudinal Yield Strength (MPa) | Longitudinal Tensile Strength (MPa) | Longitudinal Elongation (%) | Transverse Performance (Tensile Strength (MPa), Elongation (%)) | Corrosion-Breakdown Potential (Eb) vs SCE (V) | Degradation Rate (µm/y) |
|---|---|---|---|---|---|---|---|---|
| Example 1 | 300.4 | 11.56 | 921 | 980 | 36 | (776, 20) | >0.6V | 20 |
| Example 2 | 304.9 | 11.96 | 853 | 916 | 32 | (715, 18) | >0.6V | 18.6 |
| Example 3 | 328.9 | 11.51 | 832 | 897 | 38 | (693, 25) | >0.6V | 19.5 |
| Example 4 | 317.6 | 10.79 | 780 | 853 | 35 | (648, 19) | >0.6V | 20.9 |
| Example 5 | 319.3 | 10.95 | 758 | 825 | 37 | (527, 22) | >0.6V | 16.8 |
| Example 6 | 334.9 | 10.65 | 698 | 769 | 31 | (564, 17) | >0.6V | 16.5 |
| Example 7 | 327.6 | 10.41 | 671 | 750 | 34 | (549, 21) | >0.6V | 15.3 |
| Example 8 | 328 | 10.26 | 664 | 728 | 33 | (512, 23) | >0.6V | 14.2 |
| Comparative Example 1 | 329 | 10.28 | 621 | 662 | 22 | (449-483, 0) | >0.6V | 13.5 |
| Comparative Example 2 | 320 | 10.22 | 613 | 656 | 7 | (642, 0) | >0.6V | 0 |
| Comparative Example 3 | 317 | 10.16 | 529 | 602 | 2 | (328, 0) | / | / |
| Comparative Example 4 | 323 | 10.12 | 573 | 646 | 5 | (447, 0) | / | / |
| Comparative Example 5 | 315 | 10.02 | 635 | 720 | 19 | (486, 15) | >0.6V | 19.8 |

Specifically, the molybdenum-based alloys of Examples 1 to 8 and Comparative Examples 3 to 5 have been prepared by powder metallurgy or vacuum arc melting and casting in accordance with the compositions listed in Table 1. Comparative Example 1 is commercially available pure molybdenum (99.95%), and Comparative Example 2 is a commercially available (titanium-zirconium-molybdenum) TZM alloy. Relevant performance tests have been carried out on them, including elastic modulus, longitudinal yield strength, longitudinal tensile strength, longitudinal elongation, transverse tensile strength transverse elongation, corrosion resistance and degradation rate, and the test results are summarized in Table 2. Transverse tensile strength data is omitted from Table 2 due to a lack of significance.

Previous studies have shown that transverse elongation of pure molybdenum and the TZM alloy is not possible at all. In contrast, as can be seen from Tables 1 and 2, the molybdenum-based alloys prepared in accordance with Examples 1 to 8 of this invention, which contain the strengthening and toughening alloying element(s) (at least one of Ta, Ir and Pt) and purifying alloying element(s) (at least one of Sr, Ti and Ce), all possess remarkably improved transverse and longitudinal mechanical properties (tensile strength and elongation) over the commercially available pure molybdenum and TZM alloy. The results of immersion degradation tests show that the TZM alloy is non-degradable.

As shown in Table 2, the molybdenum-based alloy of Comparative Example 3 contains 0.03% of Ca. Due to "blistering" and extremely large coarse grains attributed to the presence of Ca at grain boundaries, the molybdenum-based alloy exhibits very poor plasticity. Its axial elongation is only 2%, and its radial elongation is zero. The molybdenum-based alloy of Comparative Example 4 contains 0.05% of Fe and shows relatively low yield and tensile strength. Further, cracks may develop during pressing from locations with Fe, degrading plasticity of the molybdenum-based alloy. Consequently, the molybdenum-based alloy of Comparative Example 4 has an axial elongation of only 5% and a radial elongation of 0 at break. Therefore, the presence of Ca and/or Fe is undesirable for these molybdenum-based alloys. As the mechanical properties (e.g., elongation) of the Ca-containing Mo-based alloy of Comparative Example 3 and the Fe-containing Mo-based alloy of Comparative Example 4 are far from satisfactory for preparing medical implant devices, they are not tested for immersion degradation behavior and corrosion resistance.

The molybdenum-based alloys of Example 3 and Comparative Example 5 are of similar compositions, except for the absence of polydopamine (PDA) from the alloy composition of Comparative Example 5. Because of the wetting and dispensing properties of polydopamine, the molybdenum-based alloy of Example 3 has a denser texture and contains fewer pores forming during blank sintering in powder metallurgy. Consequent, density of the molybdenum-based alloy of Example 3 (11.51 g/cm³) is higher than that of the molybdenum-based alloy of Comparative Example 5 (10.02 g/cm³) and closer to the theoretical value. Pores in the alloys may form sources of cracks during further processing or molding of them, degrading their mechanical properties. Therefore, the addition of PDA can lead to improved mechanical properties. As can be seen from Table 2, the mechanical properties (strength and elongation) of the molybdenum-based alloy of Example 3 in the different directions (longitudinal and transverse) are all superior to those of the molybdenum-based alloy of Comparative Example 5 that does not contain PDA.

The densities of Ta, Ir and Pt are 16.69 g/cm³, 22.56 g/cm³ and 21.45 g/cm³, respectively. Therefore, the inventive molybdenum-based alloys each containing at least one of Ta, Ir and Pt all have a higher density than the Mo matrix (10.28 g/cm³). As shown in Table 2, in comparison to the commercially available pure molybdenum of Comparative Example 1 and the TZM alloy of Comparative Example 2, the inventive molybdenum-based alloys each containing at least one of Ta, Ir and Pt all have a higher density, promising better radiopacity of stents made of these alloys.

A cardiovascular stent may recoil radially and/or axially. Since use of a cardiovascular stent involves crimping it onto a balloon, axial recoil would be largely balloon-dependent and thus difficult to assess. Moreover, there are no widely agreed criteria for such assessment, and axial recoil is not considered to be significantly harmful. However, during use of a cardiovascular stent, significant radial recoil is associated with a high risk of displacement or malapposition. Therefore, radial recoil assessment is important to a cardiovascular stent. According to most relevant regulations, maximum permissible radial recoil for a cardiovascular stent is below 10%, and a cardiovascular stent with less radial recoil is more desirable. Recoil of a stent is primarily dependent on the elastic modulus E of an alloy material from which the stent is fabricated. As can be seen from Table 2, the elastic modulus of each of the inventive molybdenum-based alloys of Examples 1 to 8 is lower than that of pure molybdenum, thus promising higher recoil resistance of stents made of these alloys.

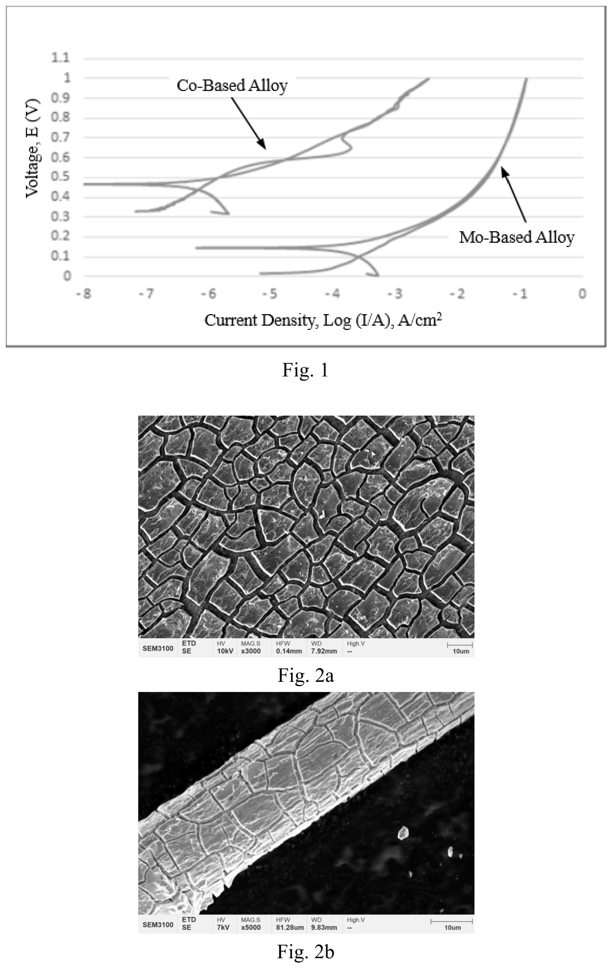

Reference is now made to Fig. 1, a schematic diagram illustrating a corrosion resistance comparison made between vascular stents made of a molybdenum-based alloy prepared in accordance with the present invention (e.g., Example 1) and a cobalt-based alloy. In particular, the vascular stents may be each post-dilated and then tested for their corrosion resistance according to a method described in YYT 0695-2008 Standard Test Method for Conducting Cyclic Potentiodynamic Polarization Measurements to Determine Corrosion Susceptibility of Small Implant Devices (ASTM F 2129-06). The vascular stent is crimped onto a balloon in the same manner as is specified in relevant standards for the implantation of this type of medical device, and the two are then crossed, as a whole, through a human body phantom three times in phosphate buffer saline (PBS) at 37 °C. The vascular stent is then dilated to its nominal size by inflating the balloon. Subsequently, the pressure in the balloon is further increased until the vascular stent is dilated to a maximum permissible size for post-dilation. For the purposes of the present invention, the vascular stent is then even further dilated using a post-dilation balloon until its struts are straightened. As a result, it is expanded to a size approximately 25% greater than the maximum permissible post-dilation size. Testing under this extreme condition can better demonstrate benefits of the present invention.

Vascular stents are Class III medical implant devices, which are required to have excellent corrosion-resistant properties, including a breakdown potential ≥ 0.6 V. Currently, mainstream medical alloys for stents available on the market are cobalt-based ones, which can provide the required corrosion-resistant properties, including a breakdown potential ≥ 0.6 V. Notably, during corrosion resistance testing, a breakdown potential can be determined at a noticeable point of inflection on a corresponding cyclic potentiodynamic polarization curve, which indicates the occurrence of pitting corrosion. Although corrosion resistance of both vascular stents made of the cobalt- and molybdenum-based alloys imparts the required breakdown potential, as can be seen from Fig. 1, the vascular stent made of the cobalt-based alloy is broken down, i.e., the cobalt-based alloy undergoes pitting corrosion, at about 0.65 V, as indicated by a noticeable point of inflection of a corresponding cyclic potentiodynamic polarization curve, while there is no point of inflection on a curve corresponding to the vascular stent made of the inventive molybdenum-based alloy even after the voltage is increased to 1 V, indicating a breakdown potential of this vascular stent above 1 V and excellent corrosion-resistant properties of the molybdenum-based alloy, which satisfy the requirements for Class III medical implant devices.

Reference is now made to Figs. 2a and 2b. Fig. 2a is a scanning electron microscope (SEM) image showing the surface morphology of a sheet of a molybdenum-based alloy according to the present invention, which has undergone uniform degradation. Fig. 2b is an SEM image showing the surface morphology of a wire of a molybdenum-based alloy according to the present invention, which has undergone uniform degradation. Specifically, the sheet (e.g., of the molybdenum-based alloy of Example 3) and wire (e.g., of the molybdenum-based alloy of Example 5) have been polished, cleaned and shaken in PBS at 37 °C and 100 revolutions per minute (rpm) for one month. As can be seen from the surface morphologies of Figs. 2a and 2b, the molybdenum-based alloy in the larger sheet-shaped form and the molybdenum-based alloy in the smaller wire-shaped form, which is compatible with the size of a stent, both break down in a uniform and steady manner into nanoscale-thick (e.g., approximately 10-µm) equiaxed flakes containing Mo, O, Ca, Mn, Na and P, which are soluble in a simulated human body fluid. In the degradation processes, no noticeable accumulation of large particles or precipitates has been observed, suggesting assured biosafety during use.

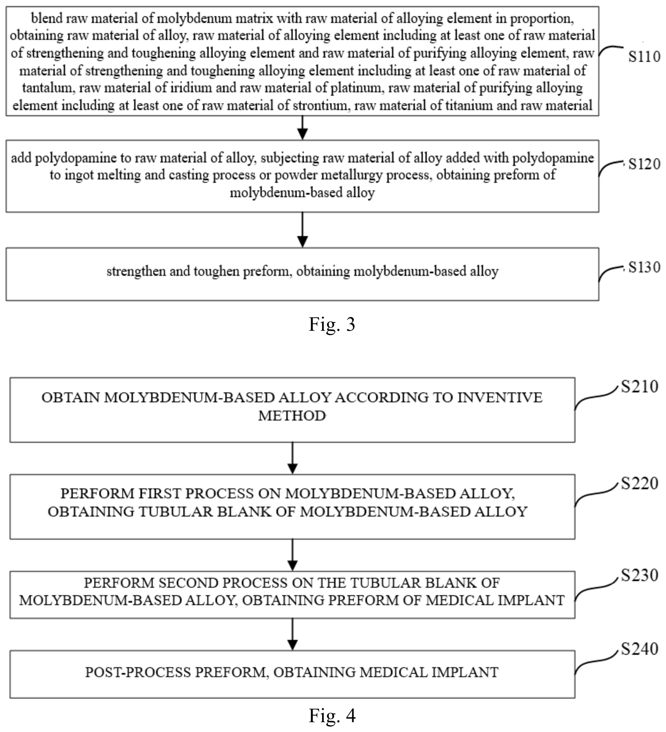

The above object of the invention is also attained by a method of preparing a molybdenum-based alloy as discussed above. Fig. 3 shows a schematic flowchart of the method according to an embodiment of the present invention. As shown in Fig. 3, the method includes the steps of:
S110) blending a raw material of a molybdenum matrix with raw material(s) of alloying element(s) at a ratio, obtaining a raw material of the alloy, the raw material(s) of the alloying element(s) including at least one of raw material(s) of strengthening and toughening alloying element(s) and raw material(s) of purifying alloying element(s), the raw material(s) of the strengthening and toughening alloying element(s) including at least one of a raw material of tantalum, a raw material of iridium and a raw material of platinum, the raw material(s) of the purifying alloying element(s) including at least one of a raw material of strontium, a raw material of titanium and a raw material of cerium;
S120) adding polydopamine to the raw material of the alloy, subjecting the raw material of the alloy added with the polydopamine to an ingot melting and casting process or a powder metallurgy process and thereby obtaining a preform of the molybdenum-based alloy; and
S130) strengthening and toughening the preform and thereby obtaining the molybdenum-based alloy.

Since the proposed molybdenum-based alloy is obtainable according to this method, the method offers all the benefits of the alloy. For details, reference is made to the above description in connection with the benefits of the proposed molybdenum-based alloy, and these are not repeated herein. According to this method, strengthening and toughening the preform can impart additionally improved mechanical properties to the resulting molybdenum-based alloy, effectively ensuring that the resulting molybdenum-based alloy exhibits high strength and high toughness.

Specifically, the raw material of the molybdenum matrix, the raw material(s) of the strengthening and toughening alloying element(s) and the raw material(s) of the purifying alloying element(s) are all 99.95% pure simple substances. The ingot melting and casting process is accomplished primarily by arc melting and casting and by electron-beam melting and casting. For more information on arc melting and casting and electron-beam melting and casting, reference is made to relevant technologies known to those skilled in the art, and further description thereof is omitted herein for the sake of brevity.

In some exemplary embodiments, the raw material of the molybdenum matrix, the raw material(s) of the strengthening and toughening alloying element(s) and the raw material(s) of the purifying alloying element(s) are provided as powders. It should be noted and will be appreciated by those skilled in the art, in the case of the ingot melting and casting process being used in the preparation of the molybdenum-based alloy, the molybdenum-based alloy, the raw material of the molybdenum matrix, the raw material(s) of the strengthening and toughening alloying element(s) and the raw material(s) of the purifying alloying element(s) may be provided either as powders, or as blocks. However, if the powder metallurgy process is used in the preparation of the molybdenum-based alloy, the molybdenum-based alloy, the raw material of the molybdenum matrix, the raw material(s) of the strengthening and toughening alloying element(s) and the raw material(s) of the purifying alloying element(s) are preferably provided as powders.

In some exemplary embodiments, before the polydopamine is added to the raw material of the alloy, the method further includes:
subjecting the raw material of the alloy to an annealing process in a reducing atmosphere or under a vacuum condition.

Annealing the raw material in a reducing atmosphere or under a vacuum condition can reduce oxides therein, achieving the purposes of removing impurities therefrom and increasing the powder's purity. Moreover, the annealing process can eliminate work hardening of the powder, increase its plasticity during pressing and compaction and stabilize crystals therein.

In particular, dopamine may be added as a molding agent (optionally further containing a conventional binder and a conventional lubricant) to the raw material of the alloy that has undergone the annealing process. When exposed to air under a weakly alkaline condition, dopamine polymerizes on the surfaces of particles in the powders, forming the polydopamine (PDA), which acts as a molding agent in a viscous, flowable form with adhesive and lubricating properties. The PDA can form nanoscale shells, which uniformly coat the surfaces of particles in the powder(s) of the alloying element(s), effectively preventing impurities such as oxide layers on the surfaces of particles in the raw materials. It also acts as a lubricant during continuous compression molding, which prevents defects such as delamination, scratches and burrs and reduces friction between the mixed alloy compact and the mold, ensuring smooth operation of the mold.

By virtue of its exceptional adhesive properties, the PDA can coat particles in the powder(s) of the alloying element(s), forming a uniform, dense nano-coating with even higher adsorption. This can prevent oxidation and impurities and enhance cohesion of particles in the powder(s). The polydopamine also provides lubrication during molding of the powders. In some exemplary embodiments, the polydopamine nano-coating has a thickness ranging from 10 nanometers to 60 nanometers. Further, the thickness of the polydopamine nano-coating may range from 10 nanometers to 50 nanometers. Further, the thickness of the polydopamine nano-coating may range from 10 nanometers to 40 nanometers. Further, the thickness of the polydopamine nano-coating may range from 10 nanometers to 30 nanometers. Further, the thickness of the polydopamine nano-coating may range from 10 nanometers to 20 nanometers.

In some exemplary embodiments, subjecting the raw material of the alloy added with the polydopamine to the powder metallurgy process and thereby obtain the preform of the molybdenum-based alloy includes:
uniformly mixing the raw material of the alloy added with the polydopamine, obtaining a mixed alloy powder;
pressing the mixed alloy powder, obtaining a mixed alloy compact; and
sintering the mixed alloy compact, obtaining the preform.

In particular, the uniform mixing of the raw material of the alloy added with the polydopamine may be accomplished mechanically or chemically in a mixer, such as a ball mill or V-shaped mixer, and the resulting mixed alloy powder may consist of coarse and fine particles and have an appropriate particle size distribution, which imparts a higher tapped density and hence reduced porosity and promotes rearrangement of particles in the powder, recrystallization and grain growth during sintering.

The mixed alloy powder may be initially loose and then subjected to a predetermined amount of pressure in a press die. In this process, the majority or all of particles in the powder are moved and rearranged, and cold welding may occur between adjacent particles, thereby forming the mixed alloy compact with predetermined dimensions, shape, density and strength. An increased amount of molding pressure can lead to fewer and smaller pores in the mixed alloy compact, and the polydopamine added to the molding agent can ensure sufficient surface adsorption and good surface adhesion of particles in the powder, which enhance cohesion of particles in the powder and help increase the density of the mixed alloy compact as much as possible. Additionally, the good dispersion properties of the polydopamine ensure a uniform density throughout the mixed alloy compact.

Further, the sintering of the mixed alloy compact may be accomplished by a solid-or liquid-state sintering process performed in a reducing atmosphere or under a vacuum condition. In the solid-or liquid-state sintering process, atoms may be thermally activated to migrate, resulting in metallurgical bonding of particles and the formation of grain boundaries therein. The alloying element(s) form(s) new compounds at grain boundaries in the matrix as a result of dissolution reactions, alloying reactions and solid-state diffusion. The sintering process may essentially consist of the following three stages: the formation of adhesion points; the formation and growth of sinter necks; and the formation and spheroidization of isolated pores. Preferably, the case of one or more low-melting-point alloying elements being added to the molybdenum-based alloy (e.g., strontium, titanium and/or cerium), the sintering of the mixed alloy compact may be accomplished by a liquid-state sintering process, in which the low-melting-point alloying elements (e.g., strontium, titanium and/or cerium) may be added as activating sintering additives to provide a liquid phase, while the refractory component (molybdenum matrix) remains in a solid phase. The liquid phase flows into pores under the action of capillary forces and preferentially dissolves sharp corners of particles in the powder, facilitating effective particle compaction. At the same time, the surfaces of solid particles are smoothed and the particles are spheroidized, reducing resistance to their rearrangement. As a result, a full-density, high-elongation molybdenum-based alloy can be obtained from the powder metallurgy process.

In some exemplary embodiments, strengthening and toughening the preform and thereby obtaining the molybdenum-based alloy includes:
subjecting the preform successively to a solid solution strengthening process, a hot deformation process and an annealing process, thereby obtaining the molybdenum-based alloy.

Through subjecting the preform successively to the solid solution strengthening, hot deformation and annealing processes, the mechanical properties of the resulting molybdenum-based alloy can be additionally improved, effectively ensuring that the alloy provides the advantages of high strength and high toughness.

In particular, the solid solution strengthening process on the preform may be a solid/solid, solid/liquid, liquid /liquid or another process. The solid/solid process can maximize uniform mixing of ions, ultimately allowing the resulting molybdenum-based alloy to have a uniform texture and significantly improved mechanical properties. The hot deformation process on the preform that follows the solid solution strengthening process may a rolling, extrusion, forging or other hot shaping process. The annealing process that follows the hot deformation process can eliminate stress generated during the hot deformation process, reducing anisotropy of the resulting molybdenum-based alloy. It should be noted and will be appreciated by those skilled in the art that, if required, one or more annealing actions may be interspersed in the hot deformation process to effectively eliminate stress generated in the middle of the process, which hinders smooth operation of the hot deformation process.

The above object of the invention is also attained by a medical implant device made of a molybdenum-based alloy as discussed above. Because of being made of a molybdenum-based alloy prepared in accordance with the present invention, the medical implant device provides the advantages of high strength, high toughness and consistent performance and is uniformly degradable, ensuring biosafety during use. In addition, this medical implant device exhibits excellent radiopacity, which brings convenience to surgeons' operation and clinical practice.

In particular, the proposed medical implant device may be a medical stent for placement into a lumen of the human body, such as a vascular or non-vascular stent. Examples of the vascular stent may include, but are not limited to, coronary stents, peripheral stents, stent grafts, intracranial stents, venous stents, stents in left atrial appendage (LAA) occluders, vascular filters, atrial and ventricular septal occluders and embolic protectors. Examples of the non-vascular stent may include, but are not limited to, esophageal stents, biliary stents and nasal stents.

The medical implant device (e.g., a vascular stent) may have a smooth or rough surface, with or without one or more grooves thereon and/or with or without one or more openings therein. Other features that may be present on the surface of the medical implant device may include, but are not limited to, any recesses or micropores for containing a drug therein. In particular, the molybdenum-based alloy may be formed into a tube, which may be then cut to create struts. After that, recesses or micropores for containing a drug therein may be formed in one or more of the struts.

In some other embodiments, an active drug coating may be directly applied to the surface of the medical implant device (e.g., vascular stent). Examples of a drug contained in the active drug coating may include those for the treatment of cardiovascular and cerebrovascular diseases, tumor diseases, intestinal diseases urinary tract diseases, etc. Without limitation, the drug may include at least one of paclitaxel, docetaxel, copper aspirinate, tacrolimus, hydroxy camptothecin, vinblastine, doxorubicin, rapamycin and rapamycin derivatives.

Some exemplary embodiments of the present invention also provide a method of preparing a medical implant device. Fig. 4 shows a schematic flowchart of the method according to an embodiment of the present invention. As shown in Fig. 4, the method includes the steps of:
S210) obtaining a molybdenum-based alloy according to a method as discussed above;
S220) performing a first process on the molybdenum-based alloy and thereby obtaining a tubular blank of the molybdenum-based alloy;
S220) performing a second process on the tubular blank and thereby obtaining a preform of the medical implant device; and
S230) post-processing the preform and thereby obtaining the medical implant device.

Since this method is based on the same inventive concept as the above discussed method, it offers all the benefits of the latter. For details, reference is made to the above description in connection with the benefits of the above discussed method, and these are not repeated herein.

In some exemplary embodiments, performing the first process on the molybdenum-based alloy and thereby obtaining the tubular blank includes:
subjecting the molybdenum-based alloy to hot piercing, hot extrusion or mechanical piercing, thereby obtaining the tubular blank.

In some exemplary embodiments, performing the second process on the tubular blank and thereby obtaining the preform includes:
subjecting the tubular blank successively to a drawing process, a stress relief annealing process and a laser cutting process, thereby obtaining the preform.

The stress relief annealing process that follows the drawing process can effectively eliminate stress generated during the drawing process, thereby reducing anisotropy of the resulting medical implant device and enhancing its plasticity.

In some exemplary embodiments, post-processing the preform and thereby obtaining the medical implant device includes:
subjecting the preform successively to a pickling process, a heat treatment process and a polishing process, thereby obtaining the medical implant device.

In particular, the pickling process can remove cutting debris, a heat-affected layer and contaminants from a surface of the preform. The heat treatment process can eliminate stress generated during previous processing of the preform, reducing anisotropy of the resulting medical implant device and enhancing its plasticity. The polishing process (e.g., electrochemical polishing) can remove an oxide layer from a surface of the preform and make the surface glossy and smooth. This can reduce surface roughness of the resulting medical implant device, reducing the risks of thrombosis and tissue reactions after it is implanted and enabling it to be sized as designed.

As discussed above, the molybdenum-based alloy, medical implant device and methods proposed herein offer the following advantages over the prior art:
1) The presence of polydopamine in the molybdenum-based alloy ensures sufficient surface adsorption and good surface adhesion of particles in a powder for preparing the alloy and provides good lubrication, addressing the problems of inadequate material compactness and uniformity that may otherwise arise in the preparation of the molybdenum-based alloy.
2) Adding the strengthening and toughening alloying element(s), such as tantalum, iridium and/or platinum, to the molybdenum-based alloy can effectively reduce yield strength of the resulting alloy, leading to an increase in its plasticity. Additionally, in doing so, the molybdenum-based alloy has an increased recrystallization temperature and behaves better at higher temperatures. The presence of the strengthening and toughening alloying element(s), such as tantalum, iridium and/or platinum, in the molybdenum-based alloy improves its room-temperature behaviors, weldability and radiation-shielding properties and fundamentally addresses the problems of room-temperature embrittlement and anisotropy. That is, the alloy is free of room-temperature embrittlement because of a ductile-to-brittle transition temperature much lower than room temperature. This ensures that vascular stents and other medical implant devices made of the proposed molybdenum-based alloy exhibit excellent mechanical properties when used at in-vivo temperatures.
3) Through adding the purifying alloying element(s) such as strontium, titanium and/or cerium to the molybdenum-based alloy, the presence of the interstitial impurities C, N and O is reduced, enhancing the prevention of the alloy's embrittlement and anisotropy. Moreover, this strengthens and toughens the molybdenum matrix, ensuring desired transverse elongation properties of a vascular stent or another medical implant device made of the alloy for post-dilation during use.
4) The proposed molybdenum-based alloy is uniformly degradable into products with good solubility. The degradation is free of noticeable accumulation of produced large particles, suggesting assured biosafety during use.
5) The proposed molybdenum-based alloy also exhibits excellent radiopacity, addressing the issue of insufficient radiopacity with conventional degradable metals and polymers and bringing huge convenience to surgeons' operation and clinical practice.

The description presented above is merely that of a few preferred embodiments of the present invention and is not intended to limit the scope thereof in any sense. Any and all changes and modifications made by those of ordinary skill in the art in light of the above teachings fall within the scope as defined in the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A molybdenum-based alloy, comprising a molybdenum matrix, polydopamine and an alloying element, the alloying element including at least one of a strengthening and toughening alloying element and a purifying alloying element, the strengthening and toughening alloying element including at least one of tantalum, iridium and platinum, the purifying alloying element including at least one of strontium, titanium and cerium.

2. The molybdenum-based alloy according to claim 1, wherein a mass percentage of the strengthening and toughening alloying element in the molybdenum-based alloy is less than or equal to 20%.

3. The molybdenum-based alloy according to claim 2, wherein the mass percentage of the strengthening and toughening alloying element in the molybdenum-based alloy is 0.5% to 2%.

4. The molybdenum-based alloy according to claim 1, wherein a mass percentage of the purifying alloying element in the molybdenum-based alloy is less than or equal to 1.0%.

5. The molybdenum-based alloy according to claim 4, wherein the mass percentage of the purifying alloying element in the molybdenum-based alloy is 0.01% to 0.04%.

6. The molybdenum-based alloy according to claim 1, wherein a mass percentage of the polydopamine in the molybdenum-based alloy is less than or equal to 1.5%.

7. The molybdenum-based alloy according to claim 6, wherein the mass percentage of the polydopamine in the molybdenum-based alloy is 0.1% to 0.3%.

8. The molybdenum-based alloy according to claim 1, wherein a mass percentage of an impurity element in the molybdenum-based alloy is less than or equal to 0.2%, wherein the impurity element includes at least one of chromium, copper, iron, nickel, tungsten, manganese, silicon, oxygen, nitrogen, carbon and hydrogen.

9. The molybdenum-based alloy according to claim 8, wherein the mass percentage of copper, iron and nickel in the molybdenum-based alloy are each less than 20 ppm, the mass percentage of carbon is less than or equal to 0.03%; the mass percentages of hydrogen, oxygen, and nitrogen are each less than or equal to 0.02%; and the mass percentages of chromium, silicon, tungsten, and manganese are each less than or equal to 0.05%.

10. The molybdenum-based alloy according to claim 1, wherein the molybdenum-based alloy does not contain any of calcium, magnesium, sodium, sulfur, lead, bismuth, zinc, rhenium, yttrium, zirconium and tin.

11. A method of preparing a molybdenum-based alloy, for preparing the molybdenum-based alloy of any one of claims 1 to 10, comprising:
blending a raw material of a molybdenum matrix with a raw material of an alloying element in proportion, obtaining an alloy raw material, the raw material of the alloying element including at least one of a raw material of the strengthening and toughening alloying element and a raw material of the purifying alloying element, the raw material of the strengthening and toughening alloying element including at least one of a raw material of tantalum, a raw material of iridium and a raw material of platinum, the raw material of the purifying alloying element including at least one of a raw material of strontium, a raw material of titanium and a raw material of cerium;
adding polydopamine to the alloy raw material, subjecting the alloy raw material added with the polydopamine to an ingot melting and casting process or a powder metallurgy process and thereby obtaining a preform of the molybdenum-based alloy; and
strengthening and toughening the preform and thereby obtaining the molybdenum-based alloy.

12. The method of preparing the molybdenum-based alloy of claim 11, wherein the raw material of the molybdenum matrix, the raw material of the strengthening and toughening alloying element and the raw material of the purifying alloying element are all in powder form,
wherein subjecting the alloy raw material added with the polydopamine to the powder metallurgy process and thereby obtaining the preform of the molybdenum-based alloy comprises:
uniformly mixing the alloy raw material added with the polydopamine, obtaining a mixed alloy powder;
pressing the mixed alloy powder, obtaining a mixed alloy compact; and
sintering the mixed alloy compact, thereby obtaining the preform of the molybdenum-based alloy.

13. The method of preparing the molybdenum-based alloy of claim 8, wherein the polydopamine is able to polymerize on surfaces of particles in the powder of the alloy raw material, forming a polydopamine nano-coating with a thickness of 10 nanometers to 60 nanometers.

14. A medical implant device, wherein the medical implant device is made of the molybdenum-based alloy of any one of claims 1 to 10.

15. A method of preparing a medical implant device, comprising:
obtaining a molybdenum-based alloy according to the method of preparing the molybdenum-based alloy of any one of claims 11 to 13;
performing a first process on the molybdenum-based alloy, obtaining a tubular blank of the molybdenum-based alloy;
performing a second process on the tubular blank of the molybdenum-based alloy, obtaining a preform of the medical implant device; and
post-processing the preform of the medical implant device, thereby obtaining the medical implant device.
